# EUROPÄISCHE PATENTSCHRIFT

(11) **EP 1 476 594 B1**
(45) Veröffentlichungstag und Bekanntmachung des Hinweises auf die Patenterteilung: **30.08.2006**
(21) Anmeldenummer: 03706508.3
(22) Anmeldetag: 14.02.2003
(51) Int. Cl.: D02G 1/02, G01N 33/36

(54) **TEXTURIERMASCHINE**
TEXTURING MACHINE
MACHINE A TEXTURER

(30) Priorität: 20.02.2002 DE 10207086
(43) Veröffentlichungstag der Anmeldung: 17.11.2004
(73) Patentinhaber: Saurer GmbH & Co. KG, 41069 Mönchengladbach (DE)
(72) Erfinder: WORTMANN, Thomas, D-42857 Remscheid (DE); SPRINGMEIER, Frank, D-42133 Wuppertal (DE); LIEBER, Reinhard, D-45549 Sprockhövel (DE)
(74) Vertreter: Kahlhöfer, Hermann
(86) Internationale Anmeldenummer: PCT/EP2003/001486
(87) Internationale Veröffentlichungsnummer: WO 2003/071016

(56) Entgegenhaltungen:
- EP-A- 0 389 849
- EP-A- 1 172 469
- DE-A- 3 324 243
- DE-A- 10 026 942
- US-A- 5 394 334

## Beschreibung

Die Erfindung betrifft eine Texturiermaschine zum Strecktexturieren einer Vielzahl von synthetischen Fäden gemäß dem Oberbegriff des Anspruchs 1.

Eine gattungsgemäße Texturiermaschine ist aus der DE 100 26 942 A1 bekannt.

Zum Strecktexturieren einer Vielzahl von Fäden besitzen derartige Texturiermaschinen eine entsprechende Vielzahl von Bearbeitungsstellen auf. Jede der Bearbeitungsstellen enthält mehrere Prozeßaggregate wie beispielsweise Lieferwerke, Falschdralltexturieraggregate und Aufwickeleinrichtungen, durch welche der Faden innerhalb der Bearbeitungsstelle gefördert, texturiert, verstreckt und zu einer Spule aufgewickelt wird. Zum Antreiben der Prozeßaggregate sind grundsätzlich zwei unterschiedliche Varianten bekannt. Bei einer ersten Variante werden alle Prozeßaggregate einer Gruppe beispielsweise alle ersten Lieferwerke der Bearbeitungsstellen gemeinsam durch einen Antrieb synchron angetrieben. Diese Variante besitzt jedoch generell den Nachteil, daß keine individuelle Steuerung der Bearbeitungsstellen möglich ist. Zur Vermeidung derartiger Nachteile ist aus der DE 100 26 942 eine Antriebsvariante bekannt, bei welcher die Prozeßaggregate durch Einzelantriebe innerhalb der Bearbeitungsstelle angetrieben werden. Dabei werden die Einzelantriebe einer Gruppe von Prozeßaggregaten benachbarter Bearbeitungsstellen wie beispielsweise alle Einzelantriebe der ersten Lieferwerke durch einen Gruppenumrichter gesteuert. Es hat sich nun jedoch herausgestellt, daß die individuelle Ansteuerung der Bearbeitungsstellen dazu führt, daß die Einzelantriebe der Prozeßaggregate unabhängig voneinander des öfteren an- und abgeschaltet werden. Dabei muß sichergestellt sein, daß die Einzelantriebe einer Gruppe von Prozeßaggregaten im Betriebszustand jeweils gleiche Betriebsparameter wie beispielsweise Antriebsdrehzahl aufweisen.

Es ist daher Aufgabe der Erfindung, eine Texturiermaschine der eingangs genannten Art derart weiterzubilden, daß selbst nach einem Abschalten einzelner Einzelantriebe die Prozeßaggregate einer Funktionsgruppe mehrerer Bearbeitungsstellen ohne größeren Steuerungsaufwand stets in einem bestimmten Betriebszustand betrieben werden können.

Diese Aufgabe wird erfindungsgemäß dadurch gelöst, daß der elektrische Einzelantrieb des Prozeßaggregates eine Asynchroneinheit zum Anlaufen bis zu einer vorgegebenen Sollfrequenz und eine Synchroneinheit zum Einhalten der vorgegebenen Sollfrequenz aufweist.

Die Erfindung besitzt somit den Vorteil, daß die Ansteuerung der Einzelantriebe durch einen Gruppenumrichter auf einfache Art derart möglich ist, daß jedem Einzelantrieb lediglich eine Sollfrequenz aufgegeben wird. Die Sollfrequenz bildet dabei den für das Prozeßaggregat erforderlichen Betriebszustand. In dem Einzelantrieb sorgt die Asynchroneinheit dafür, daß nach dem Einschalten der Einzelantrieb unmittelbar anläuft bis die Sollfrequenz erreicht ist. Bei Erreichen der Sollfrequenz tritt die Synchroneinheit des Einzelantriebes in Aktion und verhindert, daß das Prozeßaggregat mit einer von der Sollfrequenz abweichenden Frequenz angetrieben wird. Das Prozeßaggregat gelangt somit selbsttätig in einen der Sollfrequenz entsprechenden Betriebszustand. Damit ist es möglich, durch einen Gruppenumrichter eine Vielzahl von Einzelantrieben auf einfache Weise zu steuern. Die Prozeßaggregate einer Funktionsgruppe können somit nach jedem Zuschalten im Betriebszustand mit den jeweils vorgegebenen Sollparametern sicher betrieben werden. Damit ist die Gleichbehandlung aller Fäden in den Bearbeitungsstellen gewährleistet.

Grundsätzlich können die elektrischen Einzelantriebe sowohl als Asynchronmotoren oder als Synchronmotoren ausgebildet sein. Für den Fall, daß die Asynchroneinheit des Einzelantriebes durch den Asynchronmotor gebildet wird, enthält der Asynchronmotor einen Feldmagneten als Synchroneinheit. Der Feldmagnet wird vorzugsweise durch mehrere Permanentmagnete gebildet, welche an dem Rotor des Asynchronmotors befestigt sind. Damit wird erreicht, daß der Asynchronmotor nach der Beschleunigungsphase selbsttätig die vorgegebene Sollfrequenz einhält. Der Feldmagnet stellt sicher, daß der Rotor synchron zu dem Drehfeld des Stators des Asynchronmotors läuft. Diese Weiterbildung der Erfindung ist insbesondere für Prozeßaggregate geeignet, die ein relativ hohes Anlaufmoment erfordern.

Bevorzugt wird die Synchroneinheit durch einen Synchronmotor gebildet, welcher eine Hilfswicklung am Rotor als Asynchroneinheit aufweist. Damit ist sichergestellt, daß bei Ansteuerung des Einzelantriebes mit einer fest vorgegebenen Sollfrequenz der Synchronmotor ohne Verzögerung anläuft, bis der Rotor des Synchronmotors im Einklang mit dem Drehfeld des Stators ist.

Um ein individuelles Ein- und Abschalten der Bearbeitungsstellen unabhängig voneinander zu ermöglichen, ist gemäß einer besonders vorteilhaften Weiterbildung der Erfindung jeder der Einzelantriebe der Gruppe von Prozeßaggregaten über ein steuerbares Schaltelement mit dem Gruppenumrichter verbunden. Damit kann ohne Beeinflussung benachbarter Einzelantriebe und Prozeßaggregate einer oder mehrere der dem Gruppenumrichter zugeordneten Einzelantrieb abgeschaltet werden.

Darüber hinaus ist von Vorteil, wenn jeder der Einzelantriebe einen Sensor zur Drehzahlüberwachung aufweist, der mit einem die Schaltelemente steuernden Steuergerät verbunden ist. So kann vorteilhaft durch einen Ist-Soll-Vergleich eine Überbelastung der Einzelantriebe vermieden werden.

Um beispielsweise beim Anlegen der Fäden in den Bearbeitungsstellen von einer Anlegegeschwindigkeit in eine Betriebsgeschwindigkeit umzuschalten, ist gemäß einer besonders bevorzugten Weiterbildung der Erfindung das Steuergerät und der Gruppenumrichter mit einer übergeordneten Maschinensteuerung verbunden.

Bei Verwendung mehrerer Einzelantriebe für mehrere Gruppen von Prozeßaggregaten ist den Einzelantrieben einer Gruppe von Prozeßaggregaten jeweils ein Gruppenumrichter zugeordnet. Dabei sind alle Gruppenumrichter mit der Maschinensteuerung gekoppelt. Zur Erhöhung der Flexibilität einer Texturiermaschine wird gemäß einer weiteren vorteilhaften Ausführung der Erfindung vorgeschlagen, die Vielzahl der Bearbeitungsstellen in eine oder mehrere Sektionen jeweils mehreren Bearbeitungsstellen aufzuteilen. Dabei sind die Gruppenumrichter der Sektion mit einer der Sektion zugeordneten Feldsteuerung verbunden. Die Prozeßaggregate der Bearbeitungsstellen der betreffenden Sektion können somit unabhängig von den Prozeßaggregaten der Bearbeitungsstellen der benachbarten Sektionen gesteuert werden.

Die durch Einzelantriebe angetriebenen Prozeßaggregate können vorteilhaft durch ein erstes Lieferwerk und/oder ein zweites Lieferwerk und/oder ein drittes Lieferwerk pro Bearbeitungsstelle gebildet sein. Damit ist die Möglichkeit gegeben, die Fadengeschwindigkeit sowie das Verstreckverhältnis zum Verstrecken der Fäden exakt einzustellen und zu variieren.

Die Gruppe der Prozeßaggregate, die durch Einzelantriebe angetrieben werden, können jedoch auch durch eine Treibwalze einer Aufwickeleinrichtung und/oder durch ein Falschdralltexturieraggregat pro Bearbeitungsstelle gebildet sein.

Grundsätzlich sind alle rotierend angetriebenen Prozeßaggregate geeignet, die während dem Strecktexturieren der Fäden mit einer im wesentlichen vorgegebenen Sollfrequenz betrieben werden.

Im nachfolgenden ist ein Ausführungsbeispiel einer erfindungsgemäßen Texturiermaschine unter Hinweis auf die beigefügten Zeichnungen näher beschrieben.

Es stellen dar
- Fig. 1: eine schematische Seitenansicht eines ersten Ausführungsbeispiels einer erfindungsgemäßen Texturiermaschine
- Fig. 2: schematisch ein Ausschnitt einer Draufsicht eines weiteren Ausführungsbeispiels einer Texturiermaschine
- Fig. 3: schematisch ein Ausführungsbeispiel eines Einzelantriebes für ein Lieferwerk
- Fig. 4: schematisch ein weiteres Ausführungsbeispiel eines Einzelantriebes für ein Lieferwerk
- Fig. 5: ein Ausführungsbeispiel eines Einzelantriebes für eine Treibwalze einer Aufwickeleinrichtung

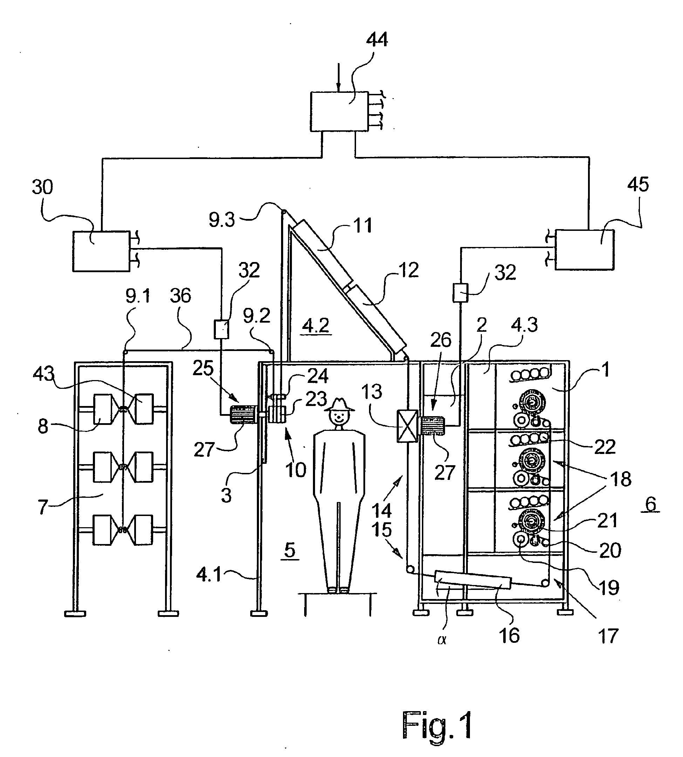

In Fig. 1 ist schematisch ein erstes Ausführungsbeispiel der erfindungsgemäßen Texturiermaschine gezeigt. Die Texturiermaschine besteht aus einen Einlaufinodul 3, einem Prozeßmodul 2 und einem Aufwickelmodul 1, die in einem Maschinengestell mit den Gestellteilen 4.1, 4.2 und 4.3 angeordnet sind. Das Einlaufmodul 3 wird durch das Gestellteil 4.1 und das Prozeßmodul 2 und das Aufwickelmodul 1 durch das Gestellteil 4.3 getragen. Das Gestellteil 4.1 und das Gestellteil 4.3 sind durch ein Gestellteil 4.2 verbunden, welches oberhalb des Einlaufmoduls 3 und des Prozeßmoduls 2 angeordnet ist. Zwischen dem Prozeßmodul 2 und dem Einlaufmodul 3 ist unterhalb des Gestellteils 4.2 ein Bediengang 5 gebildet. In dem Gestellteil 4.2 ist auf der zu dem Bediengang 5 hingewandten Seite das Prozeßmodul 2 und auf der gegenüberliegenden Seite das Aufwickelmodul 1 angeordnet. Längs des Aufwickelmoduls 1 ist ein Doffgang 6 vorgesehen. Die Texturiermaschine weist in Längsrichtung - in der Fig. 1 ist die Zeichnungsebene gleich der Querebene - eine Vielzahl von Bearbeitungsstellen auf, für jeweils einen Faden pro Bearbeitungsstelle. Die Aufwickeleinrichtungen 18 nehmen eine Breite von drei Bearbeitungsstellen ein. Daher sind jeweils drei Aufwickeleinrichtungen 18 - hierauf wird später eingegangen - in einer Säule übereinander in dem Aufwickelmodul 1 angeordnet.

In der in Fig. 1 dargestellten Ansicht sind in dem Einlaufmodul 1 und dem Prozeßmodul 2 jeweils die Prozeßaggegate einer Bearbeitungsstelle gezeigt. Jede Bearbeitungsstelle weist somit mehrere in einem Fadenlauf hintereinander angeordnete Prozeßaggregate 10, 11, 12,13,14,15,16,17 und 18 auf.

Eine erste Gruppe der Prozeßaggregate wird durch das erste Lieferwerk 10 pro Bearbeitungsstelle gebildet, das an dem Einlaufmodul 3 befestigt ist. Die benachbarten ersten Lieferwerke der benachbarten Bearbeitungsstellen sind nebeneinander (hier nicht dargestellt) angeordnet, wobei jedem ersten Lieferwerk 10 eine Vorlagespule 8 in dem Gatter 7 zugeordnet ist. Neben der Vorlagenspule 8 ist pro Bearbeitungsstelle eine Reservespule 43 in dem Gatter 7 vorgesehen. Pro Bearbeitungsstelle wird ein Faden 36 über mehrere Umlenkfadenführer 9.1 und 9.2 durch das erste Lieferwerk 10 abgezogen.

Anhand des Fadenlaufs des Fadens 36 werden nachfolgend die weiteren Prozeßaggregate einer Bearbeitungsstelle beschrieben. In Fadenlaufrichtung hinter dem ersten Lieferwerk 10 befindet sich eine langgestreckte Primärheizeinrichtung 11, durch welche der Faden 36 läuft, wobei der Faden 36 auf eine bestimmte Temperatur erwärmt wird. Die Primärheizeinrichtung 11 könnte dabei als Hochtemperaturheizer ausgeführt sein, bei dem die Heizoberflächentemperatur über 300 °C liegt. In Fadenlaufrichtung hinter der Primärheizeinrichtung 11 ist eine Kühleinrichtung 12 vorgesehen. Die Primärheizeinrichtung 11 und die Kühleinrichtung 12 sind in einer Ebene hintereinander angeordnet und werden durch das Gestellteil 4.2 oberhalb des Bediengangs 5 gehalten. Im Eingangsbereich der Primärheizeinrichtung 11 ist eine Umlenkrolle 9.3 angeordnet, so daß der Faden 36 den Bediengang 5 in einem V-förmigen Fadenlauf überquert.

Auf der zum Einlaufmodul 3 gegenüberliegenden Seite des Bediengangs 5 ist das Prozeßmodul 2 an dem Gestellteil 4.3 angeordnet. Das Prozeßmodul 2 trägt in Fadenlaufrichtung untereinander ein Falschdralltexturieraggregat 13, ein zweites Lieferwerk 14 und ein drittes Lieferwerk 15. Dabei wird der Faden 36 vom Ausgang der Kühleinrichtung 12, die vorzugsweise durch eine Kühlschiene oder ein Kühlrohr gebildet wird, zu dem Falschdralltexturieraggregat 13 geführt. Das Falschdralltexturieraggregat 13, das beispielsweise durch mehrere sich überlappende Friktionsscheiben gebildet sein kann, wird durch den Falschdrallantrieb 26 angetrieben. Der Falschdrallantrieb 26 ist als ein Einzelantrieb 27 ausgebildet, der ebenfalls an dem Prozeßmodul 2 angebracht ist.

Durch das zweite Lieferwerk 14 wird der Faden 36 aus der Falschdrallzone abgezogen, die sich zwischen dem Falschdralltexturieraggregat 13 und dem ersten Lieferwerk 10 bildet. Das zweite Lieferwerk 14 und das erste Lieferwerk 10 werden zum Verstrecken des Fadens 36 in der Falschdrallzone mit einer Differenzgeschwindigkeit angetrieben.

Unterhalb des zweiten Lieferwerks 14 ist das dritte Lieferwerk 15 angeordnet, welches den Faden 36 unmittelbar in eine Sekundärheizeinrichtung 16 führt. Die Sekundärheizeinrichtung 16 ist hierzu an der Unterseite des Gestellteils 4.3 und somit unterhalb des Prozeßmoduls 2 und des Aufwickelmoduls 1 angeordnet. Die Sekundärheizeinrichtung 16 bildet den Fadenübergang von dem Prozeßmoduls zu dem Aufwickelmodul 1. Durch die Integration des Prozeßmoduls 2, der Sekundärheizeinrichtung 16 und des Aufwickelmoduls 1 in dem Gestellteil 4.3 wird ein sehr kurzer Fadenlauf realisiert, der im wesentlichen U-förmig ausgebildet ist. Auf der Unterseite des Aufwickelmoduls 1 ist dazu ein viertes Lieferwerk 17 angeordnet, welches unmittelbar den Faden 36 aus der Sekundärheizeinrichtung 16 abzieht und nach Umlenkung des Fadens 36 zu der Aufwickeleinrichtung 18 führt. Das dritte Lieferwerk 15 und das vierte Lieferwerk 17 werden mit einer derartigen Differenzgeschwindigkeit angetrieben, daß eine Schrumpfbehandlung des Fadens 36 innerhalb der Sekundärheizeinrichtung 16 möglich ist. Die Sekundärheizeinrichtung 16 wird hierbei durch ein diphylbeheizten Kontaktheizer gebildet. Hierzu ist die Sekundärheizeinrichtung 16 um einen Winkel α gegenüber einer Horizontalen geneigt angeordnet. Der Winkel liegt im Bereich zwischen 5 bis 45 °. Damit wird sichergestellt, daß der Faden 36 innerhalb des Heizkanals der Sekundärheizeinrichtung 16 eine gleichmäßige durch Kontakt bewirkte Erwärmung erhält.

Die Aufwickeleinrichtung 18 ist bei diesem Ausführungsbeispiel schematisch durch eine Changierung 20, eine Treibwalze 19 und eine Spule 21 gekennzeichnet. Die Aufwickeleinrichtung 18 enthält zudem ein Hülsenmagazin 22 um einen automatischen Spulenwechsel auszuführen. Die zum Auswechseln der Vollspulen erforderlichen Hilfseinrichtungen sind hier nicht näher dargestellt.

Die Lieferwerke 10, 14, 15 und 17 sind in diesem Ausführungsbeispiel identisch aufgebaut, und werden jeweils durch eine Galette 23 und eine der Galetten zugeordneten Überlaufrolle 24 gebildet. Die Galette 23 wird über einen Galettenantrieb 25 angetrieben. Die Überlaufrolle 24 ist frei drehbar gelagert, so daß der Faden 36 mit mehreren Umschlingungen über die Galette 23 und die Überlaufrolle 24 geführt wird.

Bei dem in Fig. 1 dargestellten Ausführungsbeispiel der Texturiermaschine ist der Galettenantrieb 25 des ersten Lieferwerkes 10 als ein Einzelantrieb 27 ausgebildet. Der Einzelantrieb 27, dessen Aufbau nachfolgend noch näher erläutert wird, ist über ein Schaltelement 32 mit einem Gruppenumrichter 30 gekoppelt. Dem Gruppenumrichter 30 sind ebenfalls die benachbarten Einzelantriebe der hier nicht dargestellten benachbarten ersten Lieferwerke der benachbarten Bearbeitungsstellen zugeordnet. So können beispielsweise alle Einzelantriebe der ersten Lieferwerke innerhalb der Texturiermaschine einem gemeinsamen Gruppenumrichter 30 zugeordnet sein. Der Gruppenumrichter 30 ist mit einer zentralen Maschinensteuerung 44 verbunden. Das erste Lieferwerk 10 stellt somit eine erste Funktionsgruppe von Prozeßaggregaten dar, die durch Einzelantriebe 27 innerhalb der Maschine angetrieben werden.

Eine zweite Funktionsgruppe von Prozeßaggregaten stellen die Falschdralltexturieraggregate 13 dar. Die Falschdrallantriebe 26 sind ebenfalls als Einzelantriebe 27 ausgebildet, die einem zweiten Gruppenumrichter 45 zugeordnet sind. Dabei erfolgt die Anbindung der Einzelantriebe 27 einen zweiten Gruppenumrichter 45 ebenfalls durch ein Schaltelement 32. Der zweite Gruppenumrichter 45 ist ebenfalls mit der Maschinensteuerung 44 verbunden.

Die Antriebe und Antriebssteuerung der übrigen Prozeßaggregate ist hier nicht näher erläutert und könnte beispielsweise ebenfalls durch Einzelantriebe mit Steuerung über Gruppenumrichter oder durch einzeln gesteuerte Antriebe ausgebildet sein.

Im Betrieb werden die Einzelantriebe 27 der Lieferwerke 10 und der Falschdralltexturieraggregate 13 über die zugeordneten Gruppenumrichter 30 und 45 mit einer durch die Maschinensteuerung 44 definierten Sollfrequenz gesteuert, so daß das Lieferwerk 10 eine bestimmte Umfangsgeschwindigkeit zur Förderung des Fadens 36 aufweist und so daß das Falschdralltexturieraggregat 13 ebenfalls ein zum Texturieren des Fadens erforderliche Antriebsdrehzahl erhält. Der Faden 36 wird in der Bearbeitungsstelle bekannterweise gefördert, verstreckt, texturiert und zu einer Spule 21 aufgewickelt. Für den Fall, daß in der dargestellten Bearbeitungsstelle eine Störung beispielsweise durch Fadenriß eintritt, werden die Einzelantriebe 27 des Lieferwerkes 10 und des Falschdralltexturieraggregates 13 durch die Schaltelemente 32 von dem jeweiligen Gruppenumrichter 30 oder 45 getrennt. Das erste Lieferwerk 10 und das Falschdralltexturieraggregat 13 sind ausgeschaltet. Benachbarte Bearbeitungsstellen bleiben von dieser Aktion unbeeinflußt. Die in den Gruppenumrichtern 30 und 45 zugeordneten Einzelantriebe bleiben im unveränderten Betriebszustand.

Nachdem die Störung in der Bearbeitungsstelle beseitigt ist, erfolgt über die Schaltelemente 32 ein erneutes Zuschalten zu den Gruppenumrichter 30 und 45, so daß ein Ansteuern der Einzelantriebe 27 wieder möglich ist. Den Einzelantrieben 27 wird so die Sollfrequenz aufgegeben.

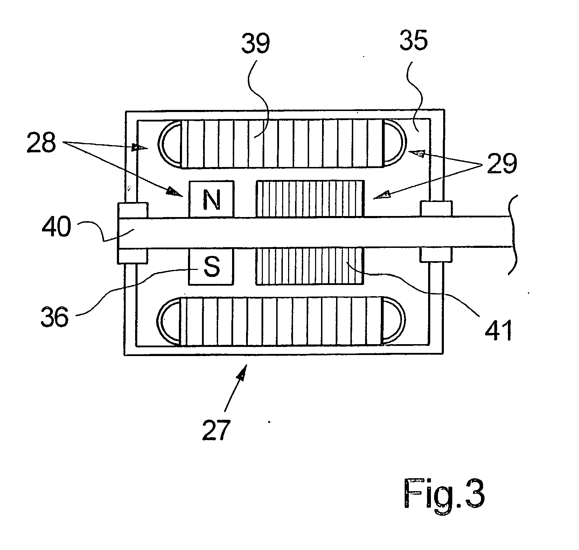

Um das An- und Abschalten sowie das Anlaufen und das Halten im Betriebszustand der Einzelantriebe 27 ohne größeren Steuerungsaufwand zu ermöglichen, besitzen die Einzelantriebe 27 jeweils eine Synchroneinheit und eine Asynchroneinheit. In Fig. 3 ist ein ersten Ausführungsbeispiel eines Einzelantriebes 27 dargestellt. Hierbei ist der Einzelantrieb 27 als ein Asynchronmotor 35 ausgebildet. Der Asynchronmotor 35 stellt somit die Asynchroneinheit 29 bestehend aus einer Statorwicklung 39 und einer Rotorwicklung 41 dar. Hierzu ist die Rotorwicklung 41 an dem Rotor 40 angebracht. Innerhalb der Statorwicklung 39 trägt der Rotor 40 einen Feldmagneten 36, der zusammen mit der Statorwicklung 39 die Synchroneinheit 28 darstellt. Der Feldmagnet 36 wird hierbei durch mehrere Permanentmagnete gebildet, die am Umfang des Rotors 40 befestigt sind. Der Rotor 40 ist an einem aus dem Motorgehäuse herausragenden Ende mit der Galette 23 des ersten Lieferwerkes 10 verbunden.

Zum Anlaufen des Asynchronmotors 35 wird über den Gruppenumrichter 30 eine Sollfrequenz aufgegeben. Nach der Bestromung der Statorwicklung 31 wird der Rotor 40 beschleunigt. Sobald die Drehfrequenz des Rotors 40 der Sollfrequenz entspricht, tritt eine Kopplung zwischen dem Drehfeld der Statorwicklung 39 und der Drehfrequenz des Rotors 40 durch den Feldmagnet 36 ein. Der Einzelantrieb 27 verhält sich im Betriebszustand entsprechend einer Synchronmaschine. Damit ist sichergestellt, daß die durch den Gruppenumrichter 30 vorgegebene Sollfrequenz selbsttätig von dem angesteuerten Einzelantrieb 28 eingestellt wird. Dies ist insbesondere für die als Lieferwerke in der Texturiermaschine angeordneten Prozeßaggregate wichtig. Somit wird in jeder Bearbeitungsstelle der Faden unter gleichen Bedingungen gefördert und verstreckt.

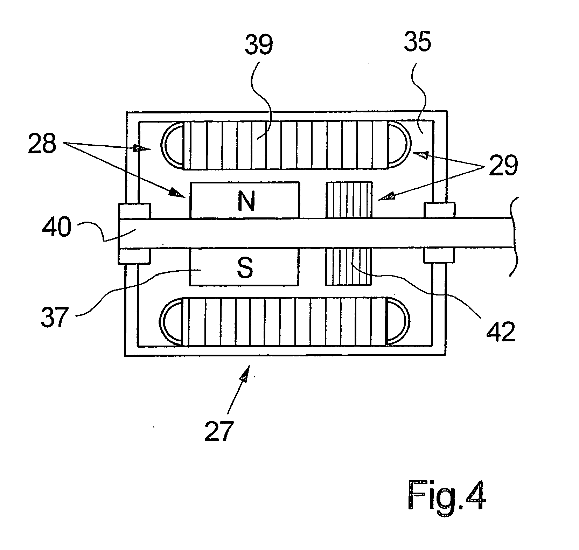

In Fig. 4 ist ein weiteres Ausführungsbeispiel eines Einzelantriebes 27 mit einer Synchroneinheit 28 und einer Asynchroneinheit 29 dargestellt. Die Bauteile gleicher Funktion sind hierbei mit identischen Bezugszeichen versehen. Die Synchroneinheit 28 wird durch ein Synchronmotor 38 gebildet. Hierzu weist der Synchronmotor 38 eine Statorwicklung 39 und einen Rotor 40 mit zumindest einem Permanentmagnet 37 auf. In diesem Fall ist die Drehfrequenz des Rotors 40 gleich der Sollfrequenz, so daß der Rotor 40 synchron mit dem Drehfeld der Statorwickler umläuft. Um nach einem Abschalten des Einzelantriebes 27 ein Anlaufen ohne Veränderung der Sollfrequenz zu ermöglichen, besitzt der Synchronmotor 38 eine Asynchroneinheit 29 auf, die durch eine Hilfswicklung 42 am Rotor 40 und der Statorwicklung 39 gebildet ist. Die Hilfswicklung 42 ist innerhalb der Statorwicklung 39 angeordnet. Somit ist sichergestellt, daß der Rotor 40 bei vorgegebener Sollfrequenz der Statorwicklung 39 beschleunigt wird.

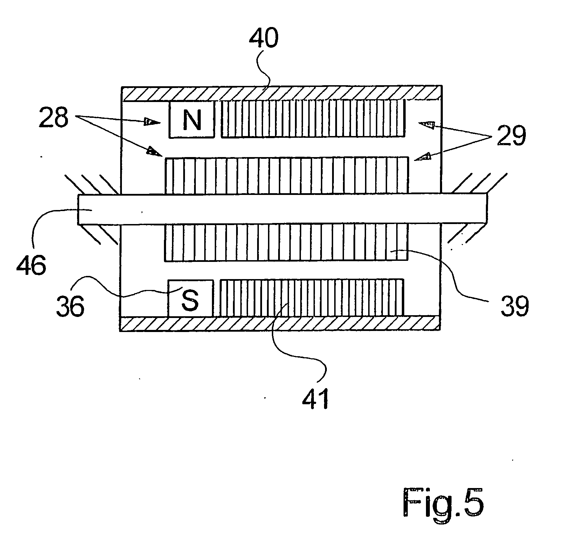

Die in den Fig. 3 und 4 gezeigten Ausführungsbeispiele des Einzelantriebes sind vorzugsweise zum Antreiben der Lieferwerke einer Texturiermaschine oder zum Antreiben eines Falschdralltexturieraggregates geeignet. In Fig. 5 ist ein weiteres Ausführungsbeispiel eines Einzelantriebes 27 gezeigt, das vorzugsweise zum Antreiben einer Treibwalze 19 in einer Aufwickeleinrichtung 18 geeignet ist. Hierzu wird der Walzenmantel der Treibwalze 19 unmittelbar durch den innerhalb der Treibwalze 19 angeordneten Einzelantrieb 27 angetrieben. Dazu beisitzt der Einzelantrieb 27 einen mantelförmigen Rotor 40. An der Innenseite des mantelförmigen Rotors 40 ist die Rotorwicklung 41 angebracht. Der Rotorwicklung 41 gegenüberliegend ist eine Statorwicklung 39 an einer feststehenden Achse 46 befestigt. Die Statorwicklung 39 erstreckt sich in axialer Richtung über die Rotorwicklung 41 hinaus um einen am mantelförmigen Rotor 40 befestigten Feldmagneten 36 zu überdecken. Der Feldmagnet 36 und die Statorwicklung 39 bilden somit die Synchroneinheit 28 des Einzelantriebes 27. Die Asynchroneinheit 29 ist hierbei durch den Aufbau als Asynchronmotor 35 gebildet. Die Funktionsweise des in Fig. 5 dargestellten Ausführungsbeispieles ist identisch zu den in Fig. 3 und 4 beschriebenen Einzelantrieben.

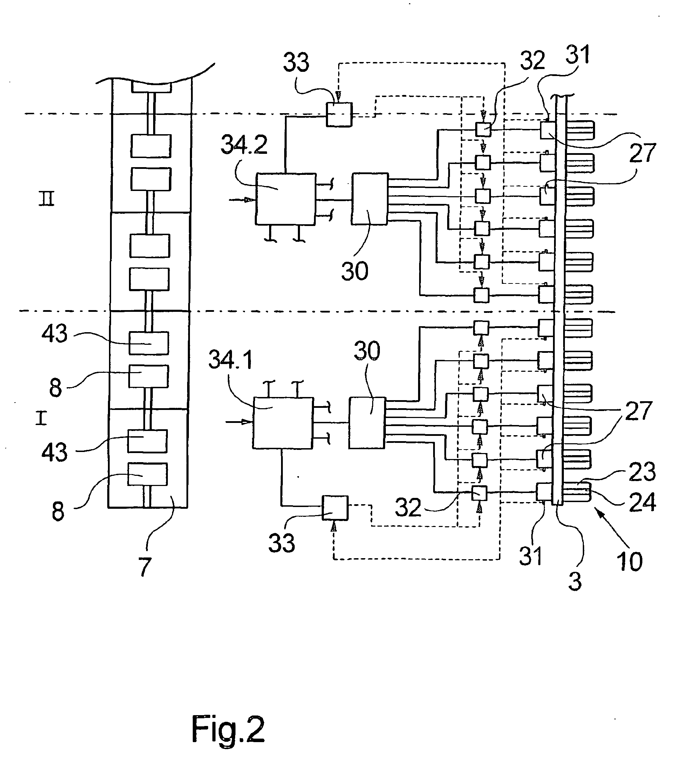

In Fig. 2 ist ein weiteres Ausführungsbeispiel einer Texturiermaschine dargestellt. Hierbei ist ein Ausschnitt einer Draufsicht des Ausführungsbeispiels gezeigt. Das Ausführungsbeispiel nach Fig. 2 ist im wesentlichen identisch zu dem vorhergehenden Ausführungsbeispiel nach Fig. 1 aufgebaut. Insoweit ist die Anordnung der Prozeßaggregate innerhalb einer Bearbeitungsstelle identisch aufgebaut, so daß zu der vorhergehenden Beschreibung Bezug genommen wird.

Bei der in Fig. 2 dargestellten Draufsicht ist nur der Fadeneinlauf der Maschine mit dem Gatter 7 und dem Einlaufinodul 3 gezeigt. Das Prozeßmodul 2 und das Aufwickelmodul 1 sind hierbei nicht dargestellt. Insgesamt sind 12 Bearbeitungsstellen nebeneinander gezeigt. Dabei sind in dem Gatter 7 jeweils die Vorlagespulen 8 von drei nebeneinander angeordneten Bearbeitungsstellen etagenmäßig übereinander angeordnet wie aus Fig. 1 ersichtlich. Der Übersicht halber ist der Fadenlauf in Fig. 2 jedoch nicht gezeigt.

An dem Einlaufmodul 3 sind die den Faden 36 von jeweils einer Vorlagenspule 8 des Gatters 7 abziehenden ersten Lieferwerke 10 nebeneinander angeordnet. Pro Bearbeitungsstelle ist ein erstes Lieferwerk 10 vorgesehen. Das erste Lieferwerk 10 besteht dabei jeweils aus einem Einzelantrieb 27, welcher mit einer Galette 23 gekoppelt ist. Der Galette 23 ist eine Überlaufrolle 24 zugeordnet.

Zur Steuerung ist der Einzelantrieb 27 über ein Schaltelement 32 mit einem Gruppenumrichter 30 verbunden. Der Gruppenumrichter 30 versorgt die Einzelantriebe 27 von insgesamt sechs Lieferwerken mehrerer Bearbeitungsstellen. Hierbei bilden jeweils sechs Bearbeitungsstellen eine Sektion, die mittels einer Feldsteuerung 34 gesteuert werden. Somit ist der Gruppenumrichter 30 mit der Feldsteuerung 34.1 der ersten Sektion I von Bearbeitungsstellen verbunden. Dementsprechend sind die Einzelantriebe 27 der Lieferwerke 10 der zweiten Sektion II über einen weiteren Gruppenumrichter 30 gesteuert, der wiederum mit der zugeordneten Feldsteuerung 34.2 gekoppelt ist.

Die Feldsteuerungen 34.1 und 34.2 sind mit weiteren Gruppenumrichtem oder Steuergeräten oder Antriebseinheiten zur Steuerung der Bearbeitungsstellen verbunden.

Den Einzelantrieben 27 einer Sektion ist desweiteren ein Steuergerät 33 zugeordnet, das mit jedem den Einzelantrieben 27 zugeordneten Schaltelementen 32 einer Sektion verbunden ist. Die Einzelantriebe 27 weisen desweiteren einen Sensor 31 auf, welche mit dem Steuergerät 33 verbunden sind. Das Steuergerät 33 ist zudem mit der Feldsteuerung 34 gekoppelt.

Die Feldsteuerungen 34.1 und 34.2 sowie weitere benachbarte Feldsteuerungen der Texturiermaschine sind mit einer zentralen Maschinensteuerung (hier nicht dargestellt) gekoppelt.

Bei der in Fig. 2 gezeigten Texturiermaschine werden im Betriebszustand die Einzelantriebe 27 der ersten Lieferwerke 10 pro Sektion durch einen Gruppenumrichter 30 mit einer vorgegebenen Sollfrequenz angesteuert. Hierzu wird dem Gruppenumrichter 30 sowie dem Steuergerät 33 durch die Feldsteuerung 34 die entsprechende Sollfrequenz, welche eine bestimmte Abzugsgeschwindigkeit der Fäden von den Vorlagespulen 8 entspricht, vorgegeben. Bei Prozeßbeginn wird jeder der Einzelantriebe 27 aufgrund der jeweils enthaltenen Asynchroneinheit beschleunigt. Sobald die Drehfrequenz des Rotors die Sollfrequenz erreicht, wird durch die Synchroneinheit der Einzelantriebe 27 an jedem der Lieferwerke 10 eine vorgegebene Umfangsgeschwindigkeit eingestellt.

Für den Fall, daß bei einem der Einzelantriebe 27 eine Störung auftritt, die eine unzulässige Abweichung von der Sollfrequenz zeigt, wird über den Sensor 31 dem Steuergerät 33 und dem Schaltelement 32 der betreffende Einzelantrieb 27 vom Gruppenumrichter 30 abgeschaltet. Hierzu findet in dem Steuergerät 33 ein Vergleich zwischen dem von dem Sensor 31 signalisierten Ist-Zustand mit einem von der Feldsteuerung 34 aufgegebenen Soll-Zustand statt. Bei unzulässiger Abweichung zwischen dem Ist-Zustand vom Soll-Zustand wird das betreffende Schaltelement 32 durch das Steuergerät 33 aktiviert. Hierbei erfolgt ein gegenseitiger Informationsaustausch zwischen dem Steuergerät 33 und der Feldsteuerung 34. Sobald die Störung beseitigt ist, wird über das Steuergerät 33 das entsprechende Schaltelement zur Ansteuerung des Einzelantriebes aktiviert. Die durch den Gruppenumrichter 30 benachbarten Einzelantriebe 27 bleiben dabei in ihrer Steuerung unbeeinflußt. Durch die in den Einzelantrieben 27 ausgebildeten Synchroneinheiten und Asynchroneinheiten ist ein selbständiges Anlaufen und Einstellen der gewünschten Umfangsgeschwindigkeiten an den Lieferwerken gewährleistet. Damit wird eine hohe Gleichmäßigkeit der Fadenbehandlung in jeder der Bearbeitungsstellen der Texturiermaschine erreicht, ohne die Flexibilität in der Ansteuerung der einzelnen Bearbeitungsstellen zu mindern. Damit vereinigt die erfindungsgemäße Texturiermaschine die Vorteile eines Gruppenantriebes für Prozeßaggregate gleicher Funktion mit den Vorteilen einer mit einzeln angetriebenen Prozeßaggregaten gesteuerten Bearbeitungsstelle.

### Bezugszeichenliste

- 1: Aufwickelmodul
- 2: Prozeßmodul
- 3: Einlaufinodul
- 4: Gestellteil
- 5: Bediengang
- 6: Doffgang
- 7: Gatter
- 8: Vorlagespule
- 9: Umlenkrolle
- 10: Erstes Lieferwerk
- 11: Primärheizeinrichtung
- 12: Kühleinrichtung
- 13: Falschdralltexturieraggregat
- 14: Zweites Lieferwerk
- 15: Drittes Lieferwerk
- 16: Sekundärheizeinrichtung
- 17: Viertes Lieferwerk
- 18: Aufwickeleinrichtung
- 19: Treibwalze
- 20: Changierung
- 21: Spule
- 22: Hülsenmagazin
- 23: Galette
- 24: Überlaufrolle
- 25: Galetteneinheit
- 26: Falschdrallantrieb
- 27: Einzelantrieb
- 28: Synchroneinheit
- 29: Asynchroneinheit
- 30: Gruppenumrichter
- 31: Sensor
- 32: Schaltelement
- 33: Steuergerät
- 34: Feldsteuerung
- 35: Asynchronmotor
- 36: Feldmagnet
- 37: Permanentmagnet
- 38: Synchronmotor
- 39: Statorwicklung
- 40: Rotor
- 41: Rotorwicklung
- 42: Hilfswicklung
- 43: Reservespule
- 44: Maschinensteuerung
- 45: Zweiter Gruppenumrichter
- 46: Achse

## Patentansprüche

1. Texturiermaschine zum Strecktexturieren einer Vielzahl von synthetischen Fäden mit einer entsprechenden Vielzahl von Bearbeitungsstellen, wobei die Fäden in den Bearbeitungsstellen parallel durch mehrere Prozeßaggregate (1013, 14, 15, 18) gefördert, texturiert, verstreckt und aufgewickelt werden, wobei zumindest einem der Prozeßaggregate (10) innerhalb der Bearbeitungsstelle ein elektrischer Einzelantrieb (27) zugeordnet ist und wobei die Einzelantriebe (27) der Gruppe von Prozeßaggregate (10) benachbarter Bearbeitungsstellen durch einen Gruppenumrichter (30) steuerbar sind, **dadurch gekennzeichnet, daß** der elektrische Einzelantrieb (27) des Prozeßaggregates (10) eine Asynchroneinheit (29) zum Anlaufen bis zu einer vorgegeben Sollfrequenz und eine Synchroneinheit (28) zum Einhalten der vorgegebenen Sollfrequenz aufweist.

2. Texturiermaschine nach Anspruch 1, **dadurch gekennzeichnet, daß** die Asynchroneinheit (29) durch einen Asynchronmotor (35) gebildet ist, welcher einen Feldmagneten (36) als Teil der Synchroneinheit (28) aufweist.

3. Texturiermaschine nach Anspruch 2, **dadurch gekennzeichnet, daß** der Feldmagnet (36) durch einen oder mehrerer Permanentmagnete gebildet ist, welche an dem Rotor (40) des Asynchronmotors (35) angeordnet sind.

4. Texturiermaschine nach Anspruch 1, **dadurch gekennzeichnet, daß** die Synchroneinheit (28) durch einen Synchronmotor (38) gebildet ist, welcher eine Hilfswicklung (42) am Rotor (40) als Teil der Asynchroneinheit (29) aufweist.

5. Texturiermaschine nach einem der Ansprüche 1 bis 4, **dadurch gekennzeichnet, daß** jeder der Einzelantriebe (27) der Gruppe von Prozeßaggregaten (10) mehrerer Bearbeitungsstellen über ein steuerbares Schaltelement (32) mit dem Gruppenumrichter (30) verbunden sind.

6. Texturiermaschine nach Anspruch 5, **dadurch gekennzeichnet, daß** jedem der Einzelantriebe (27) ein Sensor (31) zur Drehzahlüberwachung zugeordnet ist und daß die Sensoren (31) und die Schaltelemente (32) mit einem Steuergerät verbunden sind.

7. Texturiermaschine nach Anspruch 6, **dadurch gekennzeichnet, daß** das Steuergerät (33) und der Gruppenumrichter (40) mit einer den betreffenden Bearbeitungsstellen zugeordneten Steuereinheit (34) verbunden sind.

8. Texturiermaschine nach einem der Ansprüche 1 bis 7, **dadurch gekennzeichnet, daß** die Einzelantriebe (27) einer ersten Gruppe von Prozeßaggregaten (10) durch einen ersten Gruppenumrichter (30) und die Einzelantriebe (27) einer zweiten Gruppe von Prozeßaggregaten (13) durch einen zweiten Gruppenumrichter (45) steuerbar sind und daß die Gruppenumrichter (30, 45) mit der Steuereinrichtung (44) verbunden sind.

9. Texturiermaschine nach einem der vorgenannten Ansprüche, **dadurch gekennzeichnet, daß** die Vielzahl der Bearbeitungsstellen in eine oder mehrere Sektionen (I, II) mit jeweils mehreren Bearbeitungsstellen aufgeteilt sind und daß die Gruppenumrichter (30) einer der Sektionen mit einer der Sektion zugeordnete Feldsteuerung (34) verbunden sind.

10. Texturiermaschine nach einem der Ansprüche 1 bis 9, **dadurch gekennzeichnet, daß** die Gruppe der Prozeßaggregate durch ein erstes Lieferwerk (10) und/oder ein zweites Lieferwerk (14) und/oder ein drittes Lieferwerk (15) pro Bearbeitungsstelle gebildet ist.

11. Texturiermaschine nach Anspruch 10, **dadurch gekennzeichnet, daß** zumindest ein Teil der Lieferwerke (10, 14, 15) durch jeweils eine Galetteneinheit mit einer Galette (23) und einer Überlaufrolle (24) gebildet sind, wobei die Galette (23) mit dem Einzelantrieb (27) gekoppelt ist.

12. Texturiermaschine nach einem der vorgenannten Ansprüche, **dadurch gekennzeichnet, daß** die Gruppe der Prozeßaggregate durch eine Treibwalze (19) einer Aufwickeleinrichtung (18) pro Bearbeitungsstelle gebildet ist.

13. Texturiermaschine nach einem der vorgenannten Ansprüche, **dadurch gekennzeichnet, daß** die Gruppe der Prozeßaggregate durch ein Falschdralltexturieraggregat (13) pro Bearbeitungsstelle gebildet ist.

## Claims

1. Texturing machine for draw texturing a plurality of synthetic filament yarns with a corresponding plurality of processing stations, wherein the yarns in the processing stations are parallel advanced, textured, drawn, and wound by a plurality of processing units (10, 13, 14, 15, 18), wherein within the processing station at least one of the processing units (10) is associated with an electric individual drive (27), and wherein the individual drives (27) of the group of processing units (10) of adjacent processing stations are controllable by a group frequency changer (30), **characterized in that** the electric individual drive (27) of the processing unit (10) comprises an asynchronous unit (29) for starting up to a predetermined desired frequency and a synchronous unit (28) for maintaining the predetermined desired frequency.

2. Texturing machine of claim 1, **characterized in that** the asynchronous unit (29) is formed by an asynchronous motor (35), which includes a field magnet (36) as a part of the synchronous unit (28).

3. Texturing machine of claim 2, **characterized in that** the field magnet (36) is formed by one or more permanent magnets, which are arranged on the rotor (40) of the asynchronous motor (35).

4. Texturing machine of claim 1, **characterized in that** the synchronous unit (28) is formed by a synchronous motor (38), which includes an auxiliary winding (42) on the rotor (40) as a part of the asynchronous unit (29).

5. Texturing machine of one of claims 1 to 4, **characterized in that** each of the individual drives (27) of the group of processing units (10) of a plurality of processing stations connects via a controllable switching element (32) to the group frequency changer (30).

6. Texturing machine of claim 5, **characterized in that** each of the individual drives (27) is provided with a sensor (31) for monitoring the rotational speed, and that the sensors (31) and the switching elements (32) connect to a control unit.

7. Texturing machine of claim 6, **characterized in that** the control unit (33) and the group frequency changer (30) connect to a control unit (34), which is associated to the respective processing stations.

8. Texturing machine of one of claims 1 to 7, **characterized in that** the individual drives (27) of a first group of processing units (10) are controllable by a first group frequency changer (30), and the individual drives (27) of a second group of processing units (13) by a second group frequency changer (45), and that the group frequency changers (30, 45) connect to a control system (44).

9. Texturing machine of one of the foregoing claims, **characterized in that** the plurality of the processing stations are divided into one of more sections (I, II) with respectively a plurality of processing stations, and that the group frequency changers (30) of one of the sections connect to a field control system (34) associated to the section.

10. Texturing machine of one of claims 1 to 9, **characterized in that** for each processing station, the group of processing units is formed by a first feed system (10) and/or a second feed system (14) and/or a third feed system (15).

11. Texturing machine of claim 10, **characterized in that** at least one portion of the feed systems (10, 14, 15) is each formed by a godet unit with a godet (23) and a guide roll (24), with the godet (23) being coupled with the individual drive (27).

12. Texturing machine of one of the foregoing claims, **characterized in that** for each processing station, the group of processing units is formed by a drive roll (19) of a takeup device (18).

13. Texturing machine of one of the foregoing claims, **characterized in that** for each processing station, the group of processing units is formed by a false twist texturing unit (13).

## Revendications

1. Machine à texturer pour la texturation par étirage d'une multiplicité de fils synthétiques avec une multiplicité correspondante de postes de traitement, dans quel cas les fils dans les postes de traitement sont avancés, texturés, étirés et enroulés parallèlement par une pluralité d'agrégats de processus (10, 13, 14, 15, 18), dans quel cas à l'intérieur du poste de traitement un entraînement individuel électrique (27) est associé à au moins un des agrégats de processus (10) et dans quel cas les entraînements individuels (27) du groupe d'agrégats de processus (10) de postes de traitement adjacents peuvent être commandés par un convertisseur de groupe (30), **caractérisée en ce que** l'entraînement individuel électrique (27) de l'agrégat de processus (10) a une unité asynchrone (29) pour démarrer jusqu'à une fréquence prédéterminée désirée et une unité synchrone (28) pour maintenir la fréquence prédéterminée désirée.

2. Machine à texturer selon la revendication 1, **caractérisée en ce que** l'unité asynchrone (29) est formée par un moteur asynchrone (35) qui a un inducteur (36) comme une partie de l'unité synchrone (28).

3. Machine à texturer selon la revendication 2, **caractérisée en ce que** l'inducteur (36) est formé par un ou plusieurs aimants permanents qui sont agencés sur le rotor (40) du moteur asynchrone (35).

4. Machine à texturer selon la revendication 1, **caractérisée en ce que** l'unité synchrone (28) est formée par un moteur synchrone (38) qui a un enroulement auxiliaire (42) sur le rotor (40) comme une partie de l'unité asynchrone (29).

5. Machine à texturer selon l'une des revendications 1 à 4, **caractérisée en ce que** chacun des entraînements individuels (27) du groupe d'agrégats de processus (10) d'une pluralité de postes de traitement est connecté au convertisseur de groupe (30) via un élément de commutation (32) pouvant être commandé.

6. Machine à texturer selon la revendication 5, **caractérisée en ce qu'**un capteur (31) pour le monitorage de la vitesse de rotation est associé à chacun des entraînements individuels électriques (27) et **en ce que** les capteurs (31) et les éléments de commutation (32) sont reliés à un appareil de commande.

7. Machine à texturer selon la revendication 6, **caractérisée en ce que** l'appareil de commande (33) et le convertisseur de groupe (40) sont reliés à une unité de commande (34) associée aux postes de traitements respectifs.

8. Machine à texturer selon l'une des revendications 1 à 7, **caractérisée en ce que** les entraînements individuels (27) d'un premier groupe d'agrégats de processus (10) peuvent être commandés par un premier convertisseur de groupe (30) et les entraînements individuels (27) d'un deuxième groupe d'agrégats de processus (13) peuvent être commandés par un deuxième convertisseur de groupe (45) et **en ce que** les convertisseurs de groupe (30, 45) sont reliés au dispositif de commande (44).

9. Machine à texturer selon l'une des revendications précitées, **caractérisée en ce que** la multiplicité des postes de traitement sont divisés en une ou en plusieurs sections (I, II) avec respectivement une pluralité de postes de traitement et **en ce que** les convertisseurs de groupe (30) d'une des sections sont reliés à un système de commande de champ (34).

10. Machine à texturer selon l'une des revendications 1 à 9, **caractérisée en ce que** le groupe des agrégats de processus est formé par un premier dispositif d'alimentation (10) et/ou par un deuxième dispositif d'alimentation (14) et/ou par un troisième dispositif d'alimentation (15) par poste de traitement.

11. Machine à texturer selon la revendication 10, **caractérisée en ce qu'**au moins une partie des dispositifs d'alimentation (10, 14, 15) est formée par respectivement une unité de galette avec une galette (23) et un rouleau de guidage (24), la galette (23) étant couplée à l'entraînement individuel (27).

12. Machine à texturer selon l'une des revendications précitées, **caractérisée en ce que** par poste de traitement le groupe des agrégats de processus est formé par un cylindre entraîneur (19) d'un dispositif d'enroulement (18).

13. Machine à texturer selon l'une des revendications précitées, **caractérisée en ce que** par poste de traitement le groupe des agrégats de processus est formé par un agrégat de texturation par fausse torsion (13).
